# EUROPEAN PATENT SPECIFICATION

(11) **EP 3 485 894 B1**
(45) Date of publication and mention of the grant of the patent: **06.01.2021**
(21) Application number: 18207237.1
(22) Date of filing: 20.11.2018
(51) Int. Cl.: A61K 36/734, A61K 36/53, A61K 36/185, A61P 25/00

(54) **COMBINATION OF OFFICINAL PLANTS AND THEIR USE IN THE TREATMENT AND/OR PREVENTION OF SLEEP DISORDERS**
KOMBINATION VON OFFIZINELLEN PFLANZEN UND DEREN VERWENDUNG BEI DER BEHANDLUNG UND/ODER PRÄVENTION VON SCHLAFSTÖRUNGEN
COMBINAISON DE PLANTES OFFICINALES ET LEUR UTILISATION POUR LE TRAITMENT ET/OU PREVENTION D'INSOMNIE

(30) Priority: 20.11.2017 IT 201700132442
(43) Date of publication of application: 22.05.2019
(73) Proprietor: Italfarmaco SpA, 20126 Milano (IT)
(72) Inventor: Pizzoni, Angelo, 28041Arona (NO) (IT); Pizzoni, Paolo, 28041Arona (NO) (IT)
(74) Representative: Viganò, Elena

(56) References cited:
- WO-A2-2006/120360
- US-A1- 2001 038 863
- POPOVIC ET AL: "Wild flora and its usage in traditional phytotherapy (Deliblato Sands, Serbia, South East Europe)", INDIAN JOURNAL OF TRADITIONAL KNOWLEDGE, NATIONAL INSTITUTE OF SCIENCE COMMUNICATION AND INFORMATION RESOURCES, NEW DELHI - INDIA, vol. 13, no. 1, 1 January 2014 (2014-01-01), pages 9-35, XP018029849,
- DATABASE WPI Week 201774 Thomson Scientific, London, GB; AN 2017-56470E XP002782910, & CN 107 029 151 A (CHENG M) 11 August 2017 (2017-08-11)
- DATABASE WPI Week 200757 Thomson Scientific, London, GB; AN 2007-588551 XP002782911, & CN 1 935 150 A (HEFEI QIXING PHARM SCI & TECH CO LTD) 28 March 2007 (2007-03-28)
- DATABASE WPI Week 201624 Thomson Scientific, London, GB; AN 2015-520518 XP002782912, & CN 104 740 011 A (UNIV HANSHAN NORMAL) 1 July 2015 (2015-07-01)

## Description

### Summary of the Invention

The object of the present invention is a combination of extracts of hawthorn, lavender, and hops , and a new and original composition comprising said extract combination, and its use in the treatment and/or prevention of sleep disorders.

### Technical Background

Sleep disorders are very common and include, among others, the inability to fall asleep (insomnia), and repeated nocturnal awakenings. The causes may be the most varied, for example stressful situations, worries, poor nutrition, etc. These disorders also have serious consequences on the daytime behavior of the affected subject, such as fatigue, irritability, and difficulty in concentration. It is therefore understood that a proper night's rest is necessary to avoid repercussion on daily life and, in general, for the well-being of the organism.

To combat sleep disorders, drugs such as hypnotic sedatives, for example benzodiazepines, which have many and serious side effects are often used. In fact, such drugs can induce physical and mental dependence, and tolerance, *i.e.* reduction in response after repeated administration. Furthermore, benzodiazepines have been shown to cause cognitive impairment, and significantly increase the risk of developing senile dementia and Alzheimer's disease. Last but not least, it is important to note that the half-life of these drugs is very variable, and ranges from a few hours (for example, for triazolam and midazolam) up to 1-4 days (for diazepam, bromazepam, and others). It is therefore understood that these drugs are not a valid and safe remedy for treating sleep disorders and favoring a 7-8 hours rest.

In addition to the specific drugs mentioned above, it is common to use natural substances, such as extracts and essential oils deriving from officinal plants, which do not show the serious consequences and side effects of sedative drugs. However, the effectiveness of such natural remedies is often thwarted by the approximation of the dosages, due to poor reliability of active ingredients titers in said natural substances, as well as by the fact that the preparation of said natural remedies is generally carried out by the subject taking them, a subject that often does not have the necessary scientific skills to properly manage the treatment.

There is therefore a need to provide natural substances based preparations for the treatment of insomnia disorders, which are effective, reliable and ensure proper titration of the active ingredients contained in those substances.

### Objects of the Invention

It is an object of the present invention to provide a new combination of extracts of officinal plants, useful in the treatment of sleep disorders.

It is another object of the present invention to provide a new composition comprising said extract combination, particularly suitable for the treatment and/or prevention of sleep disorders.

These and other objects are achieved by the object of the present invention, which provides a new combination of extracts of officinal plants, and an original and effective composition for its administration, prepared by means of a particular pharmaceutical technique.

### Brief Description of the Drawings

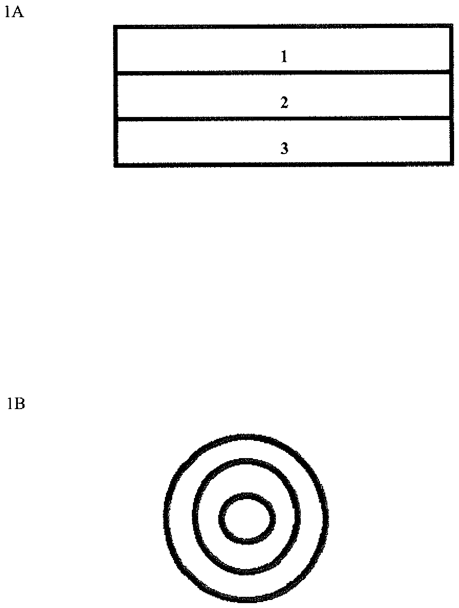

Figure 1A represents a schematic view of a section of a tablet having three overlapped layers with different release rates.
Figure 1B represents a schematic view of a section of a tablet having three concentric layers with different release rates.

### Description of the Invention

According to one of its aspects, the object of the invention relates to a combination consisting of extracts of hawthorn, lavender, and hops, advantageously said extracts being dried extracts. According to the present invention, the hawthorn is of the *Crataegus pinnatifida* species, advantageously a dry extract of this plant.

According to the present invention, the hawthorn is of the *Crataegus Oxyacantha* species, advantageously a dry extract of this plant.

According to a particularly preferred embodiment, the dry extract, titrated in vitexin, has a titer of 1-4%, advantageously about 3%.

Hawthorn is a plant drug with a cardiovasoprotective and anxiolytic action. The action of this drug on the heart and on the vessels is linked to its energetic antioxidant/anti-radical action, thanks to which it inhibits the oxidation of LDL cholesterol and lipoperoxidation, also favoring vasodilation and the activity of endogenous antioxidant enzymes.

Its sedative action occurs thanks to an increase in the intracerebral levels of GABA, an amino acid with a sedative action, interfering with the GABA-A receptor, with an effect similar to that of benzodiazepines, due to the inhibition of GABA transaminases (Biochem. Pharmacol. 40, 2227-2231, 1990; Phytother. Res. 10, suppl. 1, S 177-S 179, 1996; Pharm Biol. 48(8):924-31, 2010). Other studies show that a hawthorn extract has neuroprotective action in rats undergoing ischemia-reperfusion syndrome, mostly due to its marked antioxidant action (Int J Dev Neurosci. 2009 Dec; 27(8):799-803). A study also showed that hawthorn extract has an analgesic action antagonized by naloxone, most probably of opioid-like type (Pharm Biol. 48(8):924-31, 2010).

According to the present invention, lavender is of the *Lavandula officinalis* species, advantageously a dry extract of this plant. According to a particularly preferred embodiment, the dry extract, titrated in linalool, has a 2-6% titer, advantageously about 4%.

Lavender extract and linalool, which is its main component, do not bind to the GABA-A receptor (AANA J. 76(1):47-52, 2008).

Lavender essence reduces neurological deficits, the extent of the infarct area, the level of malondialdehyde, carbonylated proteins and ROS, and increases that of glutathione and the activity of endogenous antioxidant enzymes in the area affected by a cerebral ischemia. Lavender essence has a neuroprotective action in an animal model of cerebral ischemia-reperfusion (Molecules 17(8):9803-17, 2012).

Lavender has an action similar to that of pregabalin, by inhibiting voltage-dependent calcium channels (VDCCs) in synaptosomes of hippocampal neurons (PLoS One. 8(4):e59998). Lavender non-selectively reduces calcium uptake in different types of VDCCs, such as N-type, P/Q-type and T-type. In the hippocampus, the lavender action is exerted mainly on the N-type and P/Q-type channels.

It has also been observed that lavender essence increases striatal and hippocampal levels of serotonin and reduces its turnover, indicating that this mechanism is the basis of the sedative action of this drug. A study indicates that lavender essence stimulates striatal and hippocampal serotoninergic transmission in the rat (Nat Prod Commun. 9(7):1023-6, 2014). According to the present invention, hops is of the *Humulus lupulus* species, advantageously a dry extract of this plant. According to a particularly preferred embodiment, the dry extract, titrated in flavonoids, has a titer of 2-6%, advantageously about 4%.

Hops is also a plant drug having a sedative and sleep-promoting effect. This action is due to an increase in the intracerebral levels of GABA, an amino acid with a sedative action, interfering with the GABA-A receptor, with an effect similar to that of benzodiazepines, due to the inhibition of GABA transaminases. Hops also has a good antioxidant/anti-radical and neuro-protective action, hindering the brain damage induced by ischemia-reperfusion (Int J Dev Neurosci. 2009 Dec; 27(8):799-803).

The dried extracts of the officinal plants of the invention are known in the art and may be prepared by any method useful for the purpose.

According to a preferred embodiment, the extracts of the invention, in particular at least the hops extract, are obtained by supercritical CO₂ technology; as it is known, this technology allows to obtain high-titer and very pure extracts.

According to a preferred embodiment, in the combination of the invention, the dried extracts of hawthorn/lavender/hops, advantageously having the titers reported above, are present in a weight ratio (w/w) of 1/0.5-1/0.5-1.5, respectively, more preferably about 1/0.8/1.

The combination is particularly useful and effective to combat sleep disorders and promote adequate night rest.

These extracts have a good tolerability. In fact, none of these extracts showed any teratogenic and/or mutagenic effects neither *in vitro* nor *in vivo.*

Both acute and chronic toxicity tests in experimental animals gave a completely negative result, even for dosages ten times higher than those usually employed.

The use of the combination in the treatment of sleep disorders is a further object of the invention.

The use of the combination in the prevention of sleep disorders is a further object of the invention.

This combination has a synergistic action at the level of the Central Nervous System, acting on different receptor and signal pathways. Therefore, the combination of these three extracts guarantees an optimal synergy of action, to obtain a better sedative, anxiolytic, and antidepressant action.

Thanks to this synergy, this combination can perform its action in the treatment and/or prevention of sleep disorders at best.

The term "sleep disorders" is herein intended to indicate any sleep-related disorder, such as insomnia, intended as difficulty in falling asleep or the impossibility of falling asleep, repeated awakenings, etc.

For its use in the treatment and/or prevention of sleep disorders, the combination is preferably formulated in compositions suitable for oral administration, for example in appropriate oral dosage units, as solid dosage units, such as tablets, powders, or granulates in sachets, or in liquid dosage units, such as oral solutions or suspensions. Said composition, preferably an oral composition, more preferably a solid oral composition, comprising the combination of the invention, together with physiologically acceptable excipients and vehicles, represents a further object of the invention.

Said unitary dosage forms preferably comprise:
- 150 to 350 mg, preferably 200 to 300 mg of dry extract of hawthorn;
- 100 to 300 mg, preferably 150 to 250 mg of dry extract of lavender;
- 150 to 350 mg, preferably 200 to 300 mg of dry extract of hops;
said dry extracts preferably having the titers indicated above, together with one or more pharmaceutically acceptable excipients.

According to a preferred embodiment, the compositions of the invention comprise the combination as described herein, in combination with at least one active ingredients release modulator, advantageously a controlled release oral solid composition.

According to a preferred embodiment, the combination is contained in oral solid compositions of tablet type, advantageously controlled release ones.

According to a particularly preferred form, the combination is contained in an extended-release tablet which releases the dried extracts during the rest period, preferably a tablet having two or more layers with different release rates (rapid, intermediate and slow) of the active ingredients.

According to a preferred embodiment, the object of the invention is a multilayer tablet which releases the dried extracts differently during the rest period, comprising:
- a rapid dissolution layer containing dry extract of hawthorn;
- an intermediate dissolution layer containing dry extract of lavender; and
- a slow dissolution layer containing dry extract of hops, and optionally dry extract of lavender;
said dry extracts preferably having the titers indicated above.

More preferably, the object of the invention is a multilayer tablet comprising:
- a rapid dissolution layer containing 150 to 350 mg, preferably 200 to 300 mg of dry extract of hawthorn;
- an intermediate dissolution layer containing 70 to 250 mg, preferably 100 to 200 mg of dry extract of lavender; and
- a slow dissolution layer containing 150 to 350 mg, preferably 200 to 300 mg of dry extract of hops, and optionally 30 to 100 mg, preferably 40 to 70 mg of dry extract of lavender;
said dry extracts preferably having the titers indicated above.

A particularly preferred composition is a multilayer tablet comprising:
- a rapid dissolution layer containing 250 mg of dry extract of hawthorn;
- an intermediate dissolution layer containing 150 mg of dry extract of lavender; and
- a slow dissolution layer containing 250 mg of dry extract of hops, and 50 mg of dry extract of lavender;
said dry extracts preferably having the titers indicated above.

Herein, "multilayer tablet" is intended to indicate that the tablet is made of layers, preferably one overlapped on top of the other, as shown schematically in Figure 1A, comprising the different extracts mentioned above, or mixtures thereof.

Herein, the term "fast dissolution" is intended to indicate a dissolution time of about 1-10 minutes, for example around 5 minutes (Figure 1A, 1).

Herein, the term "intermediate dissolution" is intended to indicate a dissolution time of about 30-60 minutes, for example around 45 minutes (Figure 1A, 3).

Herein, the term "slow dissolution" is intended to indicate a dissolution time of about 4-8 hours, for example around 6 hours (Figure 1A, 2).

"Dissolution time" is intended to mean the time needed to completely dissolve the tablet in the desired conditions. Details of the dissolution assays according to the present invention are provided in the Experimental Section below.

As it is apparent, the different dissolutions indicated above will provide different release rates of active ingredients, *i.e.* extracts, as defined herein.

The compositions indicated above are preferably administered in the evening before going to bed or, in any case, just before the rest period.

The different release rates described above allow to obtain an appropriate sedation for all rest duration, preferably the night rest. The selection and dosage of the officinal plants described above, as well as the specific sequence of their release, have proved to be particularly suitable for obtaining correct sedation, without causing any toxic effects and without causing any side effects upon waking up, or during the day.

The techniques for preparing controlled-release tablets are known, and to obtain the different dissolutions mentioned above, conventional excipients known in the art may be used.

By way of example, in order to obtain a rapid release, polymers and/or techniques that prevent immediate release of the active principle should not be used, while different amounts of disintegrating agents, such as cross-linked sodium carboxymethylcellulose, sodium carboxymethyl starch, mixtures thereof, and the like are used. According to the present invention, a preferred disintegrating agent for the rapid and intermediate release layers is cross-linked sodium carboxymethylcellulose.

For the slow release layer, one or more suitable polymers may be used, such as cellulose derivatives, such as hydroxyethyl cellulose, hydroxypropyl cellulose, ethyl cellulose, cross-linked carboxymethyl cellulose; acrylic polymers; polyvinyl alcohol; polyvinylpyrrolidone; polyethylene glycols; starch; and the like. According to the present invention, a particularly preferred polymer for the slow-release layer is hydroxypropylmethylcellulose.

The person skilled in the art is certainly able to select other excipients, disintegrating agents, and polymers suitable for obtaining the different dissolutions provided for by the invention. As said, the compositions of the invention, in particular the tablets, may contain the conventionally used excipients, for example diluent agents, adsorbents, lubricants, gliders, sweeteners, dyes, etc.

For the preparation of the preferred oral solid composition according to the invention, for example a tablet, it is for example possible to use the methods known in the art. By way of example, a tablet press machine having three different hoppers, used to contain and dose the powder for the respective three layers, may be used. Once the processing parameters (weight of the three layers, compression strength, etc.) are set, the machine begins to fill the tablet die with the powder for the first layer, which forms the basis for building the tablet, and the punches, by crushing the powder according to the set parameters, allow the first layer preformation. Subsequently, the powder of the second hopper, which will fill the die already containing the first formed layer, is discharged and, thanks to the mechanical compression force and the agglomerating excipients contained in the formulation, the second layer is formed, which will then bind to the first layer; by working in the same way for the third layer, a single tablet is finally formed, which is made of the three layers with different actives release rates, as shown schematically in Figure 1A, where the rapid- and intermediate-release layers are the outermost layers (1 and 3), while the slow-release layer is sandwiched between the outer layers (2).

It is apparent that other types of tablets can be made using the officinal plants according to the present invention, for example a tablet with only two layers, having different release rates, or a multilayer tablet in which the various layers are applied concentrically on a central inner layer, as shown schematically in Figure 1B.

The use of the composition, as described above, to treat sleep disorders is a further object of the invention.

The use of the composition, as described above, to prevent sleep disorders is a further object of the invention.

Although the composition of the invention *per se* provides the solution to the problem posed, if desired or necessary, other natural extracts and/or other substances, nevertheless beneficial for the organism, may be added to the compositions of the present invention, provided that they do not contrast the effects of the combination and composition of the invention.

The invention will now be described for illustration purposes only, and in no way limitative, in the Experimental Section that follows.

### Experimental Section

### Example 1

A traditional tablet is prepared by compression of the following mixture:

| | |
|---|---|
| Hawthorn dry extract (3% vitexin) | 250 (7.5 mg vitexin) |
| Lavender dry extract (4% linalool) | 200 (8 mg linalool) |
| Hops dry extract (4% flavonoids) | 250 (4 mg flavonoids) |

Together with at least one polymer modulating the release rate.

### Example 2

### Tablet

A 1.3 grams tablet with three overlapped layers (Figure 1A) containing the following mixtures is prepared:

| **External layer, dissolving in 5 minutes** | **mg/tablet** |
|---|---|
| Hawthorn dry extract (3% vitexin) | 250 (7.5 mg vitexin) |
| Dicalcium phosphate | 37.3 |
| Microcrystalline cellulose | 118.15 |
| Magnesium stearate | 4.5 |
| Silicon dioxide | 4.5 |
| Cross-linked sodium carboxymethylcellulose | 28.8 |
| Yellow iron oxide | 6.75 |

| **External layer, dissolving in 45 minutes** | **mg/tablet** |
|---|---|
| Lavender dry extract (4% linalool) | 150 (6 mg linalool) |
| Dicalcium phosphate | 172.53 |
| Cross-linked sodium carboxymethylcellulose | 10 |
| Microcrystalline cellulose | 53.47 |
| Magnesium stearate | 4 |
| Silicon dioxide | 4 |
| Red iron oxide | 6 |

| **Internal layer, dissolving in 6 hours** | **mg/tablet** |
|---|---|
| Lavender dry extract (4% linalool) | 50 (2 mg linalool) |
| Hops dry extract (4% flavonoids) | 250 (4 mg flavonoids) |
| Dicalcium phosphate | 25-15 |
| Hydroxypropylmethylcellulose | 100-122 |
| Microcrystalline cellulose | 17-5 |
| Magnesium stearate | 4 |
| Silicon dioxide | 4 |

### Example 3

### Dissolution Test related to Hawthorn, Lavender and Hops Extracts

The dissolution test is carried out according to the following steps:
1) Development of an HPLC analysis method for each of the three extracts;
2) Dissolution test of the three extracts taken individually, in order to identify the appropriate dissolution medium (for example methanol, aqueous medium, 0.1 N HCl) with the optional addition of surfactants to the medium;
3) Dissolution test of the three-layer tablet in order to verify the matrix impact on the actual dissolution time of the three extracts (evaluating possible interferences).

### Example 4

### Multilayer Tablet Dissolution Test

According to the present invention, the dissolution test comprises 2 steps:
- Step I at acidic pH (0.1 N hydrochloric acid), at 37°C for 120 minutes, to mimic the gastric environmental conditions; and
- Step II at basic pH (phosphate buffer pH 6.0 USP), at 37°C for 60 minutes, to mimic intestinal conditions.

The multilayer composition of Example 2 was formulated so as to have specific release times for the various active ingredients, starting from the time of intake: 1^{st} external layer with release at about 5 minutes, 2^{nd} external layer with release at about 45 minutes, and 3^{rd} layer with release at about 6 hours. The dissolution test then provided for checks at 10 minutes, 50 minutes, and after 6 hours to visually assess whether the specific layers had actually dissolved.

The equipment used was a "Dissolution Tester Pharma Test, model PTWS1210, Apparatus 2" (paddle stirring element).

### Example 5

Insomnia disorders are among the most common sleep disorders, with prevalence rates ranging from 6 to 10% of the general population.

Psychophysiological insomnia is a specific type of insomnia characterized by heightened levels of physiological, cognitive, and emotional arousal that is present throughout the day.

In contrast, pre-sleep hyperarousal (*pre-sleep arousal*) is a state that occurs in a shorter period of the day, referring specifically to cognitive and somatic arousal while attempting to fall asleep. Pre-sleep arousal is hypothesized to arise from conditioning factors and maladaptive responses that are a reaction to chronically disturbed sleep or the inability to de-arouse. Pre-sleep arousal, which includes both somatic (for example, rapid heartbeat) and cognitive (for example, inability to stop racing thoughts) manifestations, which occurs at the time of sleep, can significantly delay the onset of sleep and, over time, can affect sleep quality and lead to symptoms of insomnia (Sleep Med 2014;15:1082-8; J Behav Ther Exp Psychiatry 2018;60:13-21).

Pre-sleep arousal can be measured using the PSAS scale (*Pre-Sleep Arousal Scale),* developed by Nicassio et al. (Behav Res Ther 1985;23:263-71). The PSAS scale is a 16-item self-compiled questionnaire on the state of arousal when falling asleep, on a scale ranging from 1 (never) to 5 (frequently). PSAS consists of two subscales: cognitive arousal and somatic arousal.

Tablets according to the invention are planned to be tested in the following clinical studies:
- A study of subjects with pre-sleep arousal recruited in a specialist care setting. Subjects will take the tablets for four weeks and will be evaluated for insomnia severity, restorative sleep, and variation of the PSAS score (for both cognitive and somatic subscales) versus baseline.
- A study of subjects with pre-sleep arousal in a general practice setting. Subjects will take the tablets for four weeks and will be evaluated for insomnia severity, restorative sleep, and variation of PSAS score (for both cognitive and somatic subscales) versus baseline.

## Claims

1. A combination of officinal plants consisting of extracts of hawthorn (*Crataegus pinnatifida* or *Crataegus Oxyacantha*), lavender (*Lavandula officinalis*), and hops *(Humulus lupulus).*

2. The combination according to claim 1, **characterized in that** said extracts are dry extracts and are titrated as follows:
- hawthorn titrated at 1-4% in vitexin,
- lavender titrated at 2-6% in linalool,
- hops titrated at 2-6% in flavonoids.

3. The combination according to claim 2, **characterized in that** the weight ratios of said dry extracts of hawthorn/lavender/hops are present in the combination at a rate of 1/0.5-1/0.5-1.5, respectively.

4. A composition of officinal plants comprising the extract combination according to any one of claims 1 to 3 together with physiologically acceptable excipients and vehicles.

5. The composition according to claim 4, **characterized in that** it is a solid oral composition.

6. The composition according to claims 4 or 5, **characterized in that** it comprises 150 to 350 mg, preferably 200 to 300 mg of dry extract of hawthorn; 100 to 300 mg, preferably 150 to 250 mg of dry extract of lavender; and 150 to 350 mg, preferably 200 to 300 mg of dry extract of hops.

7. The composition according to any one of claims 4 to 6, in the form of multilayer tablet **characterized in that** it comprises:
- a rapid dissolution layer containing dry extract of hawthorn;
- an intermediate dissolution layer containing dry extract of lavender; and
- a slow dissolution layer containing dry extract of hops, and optionally dry extract of lavender.

8. The composition according to claim 7, **characterized in that** it comprises
- a rapid dissolution layer containing 150 to 350 mg, preferably 200 to 300 mg of dry extract of hawthorn;
- an intermediate dissolution layer containing 70 to 250 mg, preferably 100 to 200 mg of dry extract of lavender; and
- a slow dissolution layer containing 150 to 350 mg, preferably 200 to 300 mg of dry extract of hops, and optionally 30 to 100 mg, preferably 40 to 70 mg of dry extract of lavender.

9. The composition according to any one of claims 4 to 8, further comprising at least one polymer selected from cellulose derivatives, acrylic polymers, polyvinyl alcohol, polyvinylpyrrolidone, polyethylene glycols, starch and sodium carboxymethyl starch.

10. The composition according to any one of claims 4 to 9, for its use in the treatment and/or prevention of sleep disorders.

## Patentansprüche

1. Kombination von Heilpflanzen, welche aus Extrakten of Hagedorn (Crataegus pinnatifida oder Crataegus Oxyacantha), Lavendel (Lavandula officinalis), und Hopfen (Humulus lupulus) besteht.

2. Kombination nach Anspruch 1, **dadurch gekennzeichnet, dass** die Extrakte TrockenExtrakte sind und wie folgt titriert sind:
Hagedorn titriert bei 1-4% in Vitexin,
Lavendel titriert bei 2-6% in Linalool,
Hopfen titriert bei 2-6% in Flavenoiden.

3. Kombination nach Anspruch 2, **dadurch gekennzeichnet, dass** die Gewichtsverhältnisse der Trockenextrakte von Hagedorn/Lavendel/Hopfen in der Kombination in einer Rate von 1/0,5-1/0,5-1,5 vorhanden sind.

4. Zusammensetzung von Heilpflanzen, welche die Extrakt-Kombinationen nach einem der Ansprüche 1 bis 3 zusammen mit physiologischen annehmbaren Exzipienten und Trägern umfasst.

5. Zusammensetzung nach Anspruch 4, **dadurch gekennzeichnet, dass** sie eine feste orale Zusammensetzung ist.

6. Zusammensetzung nach Anspruch 4 oder 5, **dadurch gekennzeichnet, dass** sie 150 bis 350 mg, vorzugsweise 200 bis 300 mg des Trockenextrakts von Hagedorn; 100 bis 300 mg, vorzugsweise 150 bis 250 mg des Trocken-Extrakts von Lavendel; und 150 bis 350 mg, vorzugsweise 200 bis 300 mg des Trocken-Extrakts von Hopfen umfasst.

7. Zusammensetzung nach einem der Ansprüche 4 bis 6, in der Form einer Mehrschicht-Tablette, **dadurch gekennzeichnet, dass** sie umfasst:
eine sich schnelle auflösende Schicht, die einen Trocken-Extrakt von Hagedorn enthält;
eine sich etwas langsamer auflösende Schicht, die einen Trocken-Extrakt von Lavendel enthält; und
eine sich langsam auflösende Schicht, die einen Trocken-Extrakt von Hopfen enthält, und wahlweise einen Trocken-Extrakt von Lavendel.

8. Zusammensetzung nach Anspruch 7, **dadurch gekennzeichnet, dass** sie umfasst:
eine sich schnelle auflösende Schicht, die 150 bis 350 mg, vorzugsweise 200 bis 300 mg eines Trocken-Extrakts von Hagedorn enthält;
eine sich etwas langsamer auflösende Schicht, die 70 bis 250 mg, vorzugsweise 100 bis 200 mg eines Trocken-Extrakts von Lavendel enthält; und
eine sich langsam auflösende Schicht, die 150 bis 350 mg, vorzugsweise 200 bis 300 mg vorzugsweise Hopfen enthält, und wahlweise 30 bis 100 mg, vorzugsweise 40 bis 70 mg eines Trocken-Extrakts von Lavendel.

9. Zusammensetzung nach einem der Ansprüche 4 bis 8, welche weiter mindestens ein Polymer umfasst, ausgewählt unter Cellulosederivaten, Acryl-Polymeren, Polyvinylalkohol, Polyvinylpyrrolidon, Polyethyleneglykolen, Stärke und Natriumcarboxymethylstärke.

10. Zusammensetzung nach einem der Ansprüche 4 bis 9, zur Verwendung bei der Behandlung und/oder Verhinderung von Schlafstörungen.

## Revendications

1. Combinaison de plantes officinales consistant en des extraits d'aubépine (*Crataegus pinnatifida* ou *Crataegus Oxyacantha*), de lavande (*Lavandula officinalis*), et de houblon (*Humulus lupulus*).

2. Combinaison selon la revendication 1, **caractérisée en ce que** lesdits extraits sont des extraits secs et sont titrés comme suit :
- aubépine titrée à 1-4 % de vitexine,
- lavande titrée à 2-6 % de linalool,
- houblon titré à 2-6 % de flavonoïdes.

3. Combinaison selon la revendication 2, **caractérisée en ce que** les proportions en poids desdits extraits secs d'aubépine/lavande/houblon sont présentes dans la combinaison en un rapport de 1/0,5 à 1/0,5 à 1,5, respectivement.

4. Composition de plantes officinales comprenant la combinaison d'extraits de l'une quelconque des revendications 1 à 3 conjointement avec des excipients et véhicules physiologiquement acceptables.

5. Composition selon la revendication 4, **caractérisée en ce qu'**il s'agit d'une composition solide à usage oral.

6. Composition selon la revendication 4 ou 5, **caractérisée en ce qu'**elle comprend 150 à 350 mg, de préférence 200 à 300 mg d'extrait sec d'aubépine ; 100 à 300 mg, de préférence 150 à 250 mg d'extrait sec de lavande ; et 150 à 350 mg, de préférence 200 à 300 mg d'extrait sec de houblon.

7. Composition selon l'une quelconque des revendications 4 à 6, sous la forme d'un comprimé multicouche, **caractérisée en ce qu'**elle comprend :
- une couche à dissolution rapide contenant de l'extrait sec d'aubépine ;
- une couche à dissolution intermédiaire contenant de l'extrait sec de lavande ; et
- une couche à dissolution lente contenant de l'extrait sec de houblon, et éventuellement de l'extrait sec de lavande.

8. Composition selon la revendication 7, **caractérisée en ce qu'**elle comprend
- une couche à dissolution rapide contenant 150 à 350 mg, de préférence 200 à 300 mg d'extrait sec d'aubépine ;
- une couche à dissolution intermédiaire contenant 70 à 250 mg, de préférence 100 à 200 mg d'extrait sec de lavande ; et
- une couche à dissolution lente contenant 150 à 350 mg, de préférence 200 à 300 mg d'extrait sec de houblon, et éventuellement 30 à 100 mg, de préférence 40 à 70 mg d'extrait sec de lavande.

9. Composition selon l'une quelconque des revendications 4 à 8, comprenant en outre au moins un polymère choisi parmi les dérivés de cellulose, les polymères acryliques, l'alcool polyvinylique, la polyvinylpyrrolidone, les polyéthylène glycols, l'amidon, et le carboxyméthyl-amidon sodique.

10. Composition selon l'une quelconque des revendications 4 à 9, pour son utilisation dans le traitement et/ou la prévention de troubles du sommeil.
